# EUROPEAN PATENT APPLICATION

(11) **EP 4 825 384 A1**
(43) Date of publication of application: **21.10.2026**
(21) Application number: 25861730.7
(22) Date of filing: 06.11.2025
(51) Int. Cl.: A61K 33/244, A61P 3/12

(54) **USE OF LANTHANUM OXYHYDROXIDE IN PREPARATION OF DRUG FOR TREATING HYPERPHOSPHATEMIA OR DISEASE ASSOCIATED WITH HYPERPHOSPHATEMIA**

(30) Priority: 20.02.2025 CN 202510192377
(71) Applicant: Beijing Horicin Biotechnology Co., Ltd., Beijing 100023 (CN)
(72) Inventor: AN, Ming, Beijing 100023 (CN); KUANG, Bin, Beijing 100023 (CN); ZHANG, Xu, Beijing 100023 (CN); WANG, Yan, Beijing 100023 (CN); CAO, Shuang, Beijing 100023 (CN); LV, Ning, Beijing 100023 (CN); JIANG, Xiaomei, Beijing 100023 (CN); DONG, Guangzhen, Beijing 100023 (CN); SU, Sheng, Beijing 100023 (CN)
(74) Representative: De Clercq & Partners
(86) International application number: PCT/CN2025/133054
(87) International publication number: WO 2026/174838

(57) **Abstract**

The present disclosure provides use of lanthanum oxyhydroxide in preparation of a drug for treating hyperphosphatemia or a disease associated with hyperphosphatemia, belonging to the field of pharmaceutical technology. The present disclosure provides the use of lanthanum oxyhydroxide in the preparation of a drug for treating hyperphosphatemia or a disease associated with hyperphosphatemia. In the present disclosure, lanthanum oxyhydroxide is used in the preparation of a drug for treating hyperphosphatemia or a disease associated with hyperphosphatemia. The lanthanum oxyhydroxide has a higher lanthanum content reaching 80.8%. When developed as a drug for treating hyperphosphatemia or a disease associated with hyperphosphatemia, the lanthanum oxyhydroxide allows for a reduced amount of drug, or a lower formulation strength and a smaller size of the dosage form required to bind an equivalent amount of phosphate in blood of a patient with hyperphosphatemia, which helps improve patient compliance and exhibits a good phosphorus-lowering effect.

## Description

The present application claims priority to Chinese Patent Application No. CN 202510192377.X, filed with the China National Intellectual Property Administration on April 11, 2025 and entitled "USE OF LANTHANUM OXYHYDROXIDE IN PREPARATION OF DRUG FOR TREATING HYPERPHOSPHATEMIA OR DISEASE ASSOCIATED WITH HYPERPHOSPHATEMIA", which is incorporated herein by reference in its entirety.

### TECHNICAL FIELD

The present disclosure relates to the field of pharmaceutical technology, and in particular to use of lanthanum oxyhydroxide in preparation of a drug for treating hyperphosphatemia or a disease associated with hyperphosphatemia.

### BACKGROUND

Chronic kidney disease is a common public health problem worldwide. One of its major complications is hyperphosphatemia, which is caused by the inability of the kidneys to excrete phosphate in the body, leading to an elevated phosphate level in the blood. The main symptom of hyperphosphatemia is that the phosphate level in the blood exceeds the normal range, which may be associated with secondary hyperparathyroidism, metabolic bone disease, soft tissue calcification, and cardiovascular calcification.

The treatment of hyperphosphatemia currently mainly includes dietary phosphorus intake control, phosphorus removal by dialysis, the use of phosphate binders and, if necessary, parathyroid resection, among them, for 90-95% of patients with end-stage renal disease, hyperphosphatemia is treated by taking phosphate binders to reduce intestinal phosphorus absorption.

Phosphate binders are mainly classified into two categories: traditional aluminum-containing or calcium-containing phosphate binders, and non-aluminum-containing, non-calcium-containing phosphate binders. Aluminum-containing phosphorus binders, such as aluminum hydroxide and aluminum carbonate, can cause aluminum toxicity with long-term use, leading to microcytic anemia, osteomalacia, Alzheimer's disease, etc. Calcium-containing phosphate binders, such as calcium carbonate and calcium acetate, can increase intestinal calcium absorption with long-term use, leading to hypercalcemia and causing cardiac and vascular calcification. Therefore, the ideal phosphate binder for treating hyperphosphatemia is a non-aluminum-containing, non-calcium-containing phosphate binder.

Currently, the main commercially available non-aluminum-containing, non-calcium-containing phosphate binder is a lanthanum carbonate chewable tablet. Anhydrous lanthanum carbonate has a lanthanum content of 60.7%, whereas the hydrate thereof has a lanthanum content of less than 60.7%. The relatively high formulation strength and the relatively large size of the dosage form result in poor patient compliance. Moreover, patients may experience gastrointestinal adverse reactions such as nausea and vomiting after taking lanthanum carbonate, which is also the main clinical deficiency of lanthanum carbonate as a phosphate binder, resulting in poor patient adherence. As a result, phosphorus levels in some patients could not be controlled well, and fail to reach the target level.

### SUMMARY

An object of the present disclosure is to provide use of lanthanum oxyhydroxide in preparation of a drug for treating hyperphosphatemia or a disease associated with hyperphosphatemia. In the present disclosure, lanthanum oxyhydroxide is used in the preparation of a drug for treating hyperphosphatemia or a disease associated with hyperphosphatemia, which can reduce the formulation strength and the size of the dosage form, help improve patient compliance, and exhibit a good phosphorus-lowering effect.

In order to achieve the above object of the present disclosure, the present disclosure provides the following technical solutions.

The present disclosure provides use of lanthanum oxyhydroxide in preparation of a drug for treating hyperphosphatemia or a disease associated with hyperphosphatemia.

The present disclosure provides use of at least one selected from the group consisting of lanthanum oxyhydroxide containing a lanthanum isotope and/or an oxygen isotope, a lanthanum oxyhydroxide solvate, and a double salt containing lanthanum oxyhydroxide in preparation of a drug for treating hyperphosphatemia or a disease associated with hyperphosphatemia.

In some embodiments, the disease associated with hyperphosphatemia includes chronic kidney disease, renal failure, secondary hyperparathyroidism, metabolic bone disease, soft tissue calcification or cardiovascular calcification.

In some embodiments, the lanthanum oxyhydroxide is in a form of a powder.

In some embodiments, the lanthanum oxyhydroxide includes a single crystal form or an amorphous form.

In some embodiments, the lanthanum oxyhydroxide has a surface in a smooth or rough state.

In some embodiments, the lanthanum oxyhydroxide has a porous structure.

In some embodiments, the lanthanum oxyhydroxide is prepared by a method including any one selected from the group consisting of:
method I: calcining lanthanum hydroxide and/or a lanthanum hydroxide hydrate to obtain the lanthanum oxyhydroxide;
method II: mixing an alkali reagent, water, and a lanthanum salt and/or a lanthanum salt hydrate, and subjecting a resulting mixture to metathesis reaction to obtain lanthanum hydroxide and/or a lanthanum hydroxide hydrate; and calcining the lanthanum hydroxide and/or the lanthanum hydroxide hydrate to obtain the lanthanum oxyhydroxide;
method III: mixing a dispersant, an alkali reagent, water, and a lanthanum salt and/or a lanthanum salt hydrate, and subjecting a resultant mixture to metathesis reaction to obtain lanthanum hydroxide and/or a lanthanum hydroxide hydrate; and calcining the lanthanum hydroxide and/or the lanthanum hydroxide hydrate to obtain the lanthanum oxyhydroxide;
method IV: mixing lanthanum oxide with water, and subjecting a resulting mixed material to combination reaction to obtain lanthanum hydroxide and/or a lanthanum hydroxide hydrate; and calcining the lanthanum hydroxide and/or the lanthanum hydroxide hydrate to obtain the lanthanum oxyhydroxide; and
method V: mixing lanthanum oxide, a dispersant, and water, and subjecting an obtained mixture to combination reaction to obtain lanthanum hydroxide and/or a lanthanum hydroxide hydrate; and calcining the lanthanum hydroxide and/or the lanthanum hydroxide hydrate to obtain the lanthanum oxyhydroxide.

In some embodiments, in the method I, the method II, the method III, the method IV, and the method V, the calcining is performed at a temperature independently of 200-800°C, and the calcining is performed independently for 1-72 h.

In some embodiments, in the method II and the method III, the alkali reagent independently includes an alkali metal hydroxide or aqueous ammonia; in the method III and the method V, the dispersant independently includes polyethylene glycol, a surfactant, or an ionic liquid.

In some embodiments, the aqueous ammonia has a concentration of 0.1-15.0 M; and an amount ratio of the lanthanum salt and/or the lanthanum salt hydrate to the aqueous ammonia is in a range of 1 g : 0.5-10 mL.

In some embodiments, the alkali metal hydroxide includes potassium hydroxide, sodium hydroxide, or lithium hydroxide; and a mass ratio of the lanthanum salt and/or the lanthanum salt hydrate to the alkali metal hydroxide is in a range of 1 : 0.2-5.

In some embodiments, in the method III and the method V, the lanthanum salt and/or the lanthanum salt hydrate is used as a lanthanum raw material, and a mass of the dispersant is 0.5-10.0% of a total mass of the lanthanum raw material.

In some embodiments, the polyethylene glycol is PEG400, PEG600, or PEG20000.

In some embodiments, the surfactant is polyvinylpyrrolidone or cetyltrimethylammonium bromide.

In some embodiments, the ionic liquid is an N-methyl-N-benzylmorpholinium ionic liquid.

In some embodiments, in the method II and the method III, the metathesis reaction is performed at a temperature independently of 0-200°C and a pressure independently of 0.1-2.0 MPa for a time independently of 1-24 h.

In some embodiments, in the method IV and the method V, an amount ratio of the lanthanum oxide to the water is independently in a range of 1 g : 10-30 mL.

In some embodiments, in the method IV and the method V, the combination reaction is performed at a temperature of 0-200°C and a pressure of 0.1-2.0 MPa for 1-24 h.

The present disclosure provides a drug for treating hyperphosphatemia or a disease associated with hyperphosphatemia, including an active ingredient and a pharmaceutically acceptable adjuvant, where the active ingredient is lanthanum oxyhydroxide.

The present disclosure provides a drug for treating hyperphosphatemia or a disease associated with hyperphosphatemia, including an active ingredient and a pharmaceutically acceptable adjuvant, where the active ingredient includes at least one selected from the group consisting of: a lanthanum oxyhydroxide solvate; lanthanum oxyhydroxide containing a lanthanum isotope and/or an oxygen isotope; and a double salt containing lanthanum oxyhydroxide.

In some embodiments, a content of the active ingredient in the drug is a range of 0.1-99.9 wt%.

In some embodiments, the pharmaceutically acceptable adjuvant includes a pharmaceutically acceptable carrier and/or diluent.

In some embodiments, a dosage form of the drug includes a granule, a tablet, a capsule, a chewable tablet, a powder, a suspension, or a dry suspension.

The present disclosure provides a method for treating hyperphosphatemia or a disease associated with hyperphosphatemia, including: administering an effective amount of an active ingredient to a patient suffering from hyperphosphatemia or a disease associated with hyperphosphatemia, where the active ingredient is lanthanum oxyhydroxide.

The present disclosure provides a method for treating hyperphosphatemia or a disease associated with hyperphosphatemia, including: administering an effective amount of an active ingredient to a patient suffering from hyperphosphatemia or a disease associated with hyperphosphatemia, where the active ingredient includes at least one selected from the group consisting of: a lanthanum oxyhydroxide solvate; lanthanum oxyhydroxide containing a lanthanum isotope and/or an oxygen isotope; and a double salt containing lanthanum oxyhydroxide.

The present disclosure provides use of lanthanum oxyhydroxide in preparation of a drug for treating hyperphosphatemia or a disease associated with hyperphosphatemia. In the present disclosure, lanthanum oxyhydroxide is used in the preparation of a drug for treating hyperphosphatemia or a disease associated with hyperphosphatemia. The lanthanum oxyhydroxide has a higher lanthanum content reaching 80.8%. When developed as a drug for treating hyperphosphatemia or a disease associated with hyperphosphatemia, it allows for a reduced amount of drug, or a lower formulation strength and a smaller size of the dosage form required to bind an equivalent amount of phosphate, which helps improve patient compliance and exhibits better phosphorus-lowering effects. The results of the test example of the present disclosure show that lanthanum oxyhydroxide has greater lanthanum-releasing capacity and stronger phosphate-binding ability in *in-vitro* experiments than lanthanum carbonate. In addition, the results of *in-vivo* animal experiments show that lanthanum oxyhydroxide can effectively lower serum phosphate level, suggesting that lanthanum oxyhydroxide has good development potential and value as a drug for treating hyperphosphatemia and a disease associated with hyperphosphatemia.

### BRIEF DESCRIPTION OF THE DRAWINGS

FIG. 1 shows the dissolution curves of lanthanum oxyhydroxide and lanthanum carbonate in a medium of hydrochloric acid solution with a pH value of 1.2.
FIG. 2 shows the dissolution curves of lanthanum oxyhydroxide and lanthanum carbonate in a medium of acetate buffer with a pH value of 3.0.
FIG. 3 shows the dissolution curves of lanthanum oxyhydroxide and lanthanum carbonate in a medium of acetate buffer with a pH value of 5.0.

### DETAILED DESCRIPTION OF THE EMBODIMENTS

The present disclosure provides use of lanthanum oxyhydroxide in preparation of a drug for treating hyperphosphatemia or a disease associated with hyperphosphatemia.

The present disclosure provides use of at least one selected from the group consisting of lanthanum oxyhydroxide containing a lanthanum isotope and/or an oxygen isotope, a lanthanum oxyhydroxide solvate, and a double salt containing lanthanum oxyhydroxide in preparation of a drug for treating hyperphosphatemia or a disease associated with hyperphosphatemia.

Currently, the main commercially available drug for treating hyperphosphatemia is a lanthanum carbonate chewable tablet. Anhydrous lanthanum carbonate has a lanthanum content of 60.7%, whereas a hydrate thereof has a lanthanum content of less than 60.7%. The relatively high formulation strength and the relatively large size of the dosage form result in poor patient compliance. Moreover, patients may experience gastrointestinal adverse reactions such as nausea and vomiting after taking lanthanum carbonate. In the present disclosure, lanthanum oxyhydroxide is used in the preparation of a drug for treating hyperphosphatemia or a disease associated with hyperphosphatemia. The lanthanum oxyhydroxide has a higher lanthanum content reaching 80.8%. When developed as a drug for treating hyperphosphatemia or a disease associated with hyperphosphatemia, the lanthanum oxyhydroxide allows for a reduced amount of drug, or a lower formulation strength and a smaller size of the dosage form required to bind an equivalent amount of phosphate, which helps improve patient compliance. Detailed descriptions are provided below.

As an embodiment of the present disclosure, the disease associated with hyperphosphatemia may include chronic kidney disease, renal failure, secondary hyperparathyroidism, metabolic bone disease, soft tissue calcification, or cardiovascular calcification.

As an embodiment of the present disclosure, the lanthanum oxyhydroxide may be in the form of a powder. In an embodiment, the lanthanum oxyhydroxide may include a single crystal form or an amorphous form. As an embodiment of the present disclosure, the lanthanum oxyhydroxide may have a surface in a smooth or rough state. In an embodiment, the lanthanum oxyhydroxide may have a porous structure.

The present disclosure provides a method for preparing lanthanum oxyhydroxide suitable for treating hyperphosphatemia or a disease associated with hyperphosphatemia, as described in detail below.

As an embodiment of the present disclosure, the lanthanum oxyhydroxide is prepared by a method including any one of:
method I: calcining lanthanum hydroxide and/or a lanthanum hydroxide hydrate to obtain the lanthanum oxyhydroxide;
method II: mixing an alkali reagent, water, and a lanthanum salt and/or a lanthanum salt hydrate, and subjecting a resulting mixture to metathesis reaction to obtain lanthanum hydroxide and/or a lanthanum hydroxide hydrate; and calcining the lanthanum hydroxide and/or the lanthanum hydroxide hydrate to obtain the lanthanum oxyhydroxide;
method III: mixing a dispersant, an alkali reagent, water, and a lanthanum salt and/or a lanthanum salt hydrate, and subjecting a resultant mixture to metathesis reaction to obtain lanthanum hydroxide and/or a lanthanum hydroxide hydrate; and calcining the lanthanum hydroxide and/or the lanthanum hydroxide hydrate to obtain the lanthanum oxyhydroxide;
method IV: mixing lanthanum oxide with water, and subjecting a resulting mixed material to combination reaction to obtain lanthanum hydroxide and/or a lanthanum hydroxide hydrate; and calcining the lanthanum hydroxide and/or the lanthanum hydroxide hydrate to obtain the lanthanum oxyhydroxide; and
method V: mixing lanthanum oxide, a dispersant, and water, and subjecting an obtained mixture to combination reaction to obtain lanthanum hydroxide and/or a lanthanum hydroxide hydrate; and calcining the lanthanum hydroxide and/or the lanthanum hydroxide hydrate to obtain the lanthanum oxyhydroxide.

The various preparation methods are described in detail below.

### Method I:

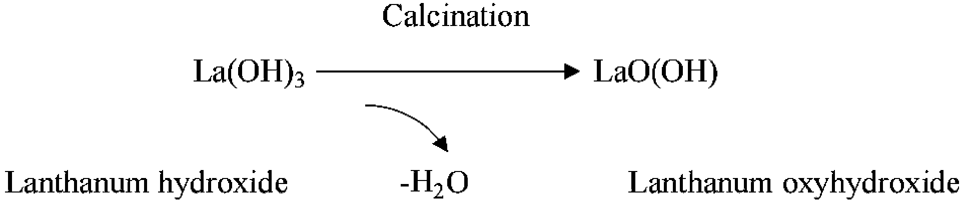

In the present disclosure, lanthanum hydroxide and/or a lanthanum hydroxide hydrate are calcinated to obtain the lanthanum oxyhydroxide.

As an embodiment of the present disclosure, the lanthanum hydroxide and/or the lanthanum hydroxide hydrate may be commercially available products well known to those skilled in the art or prepared by methods well known to those skilled in the art.

As an embodiment of the present disclosure, the calcination may be performed at a temperature of 200-800°C, further 300-500°C, and specifically 300°C, 330°C, 350°C, 380°C, 400°C, 420°C, 450°C or 500°C. In an embodiment, the calcination may be performed for 1-72 h, further 4-48 h, and specifically 4 h, 8 h, 12 h, 16 h, 18 h, 24 h, 36 h or 48 h. In an embodiment, the calcination may be performed under a protective atmosphere, which may be provided by nitrogen or an inert gas such as argon or helium.

### Method II:

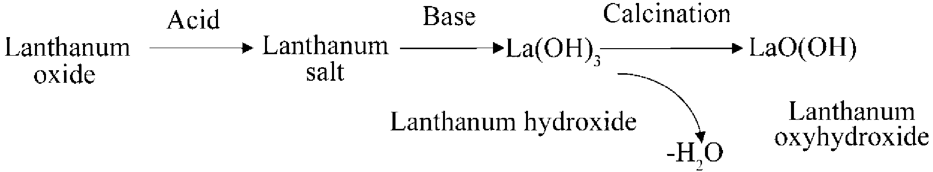

In the present disclosure, an alkali reagent, water, and a lanthanum salt and/or a lanthanum salt hydrate are mixed, and a resulting mixture is subjected to metathesis reaction to obtain lanthanum hydroxide and/or a lanthanum hydroxide hydrate; and the lanthanum hydroxide and/or the lanthanum hydroxide hydrate are calcined to obtain the lanthanum oxyhydroxide.

As an embodiment of the present disclosure, the lanthanum salt may include one or more of lanthanum chloride, lanthanum nitrate, lanthanum sulfate, and lanthanum acetate, and specifically lanthanum chloride, lanthanum nitrate, lanthanum sulfate, or lanthanum acetate. As an embodiment of the present disclosure, the lanthanum salt may be commercially available products well known to those skilled in the art or prepared by methods well known to those skilled in the art. For example, lanthanum oxide, an acid reagent, and water are mixed and reacted to obtain a lanthanum salt solution, which may then be directly mixed with an alkali reagent for metathesis reaction. The acid reagent may specifically be selected on the basis of the type of lanthanum salt desired, and may be, for example, nitric acid.

As an embodiment of the present disclosure, the alkali reagent may include an alkali metal hydroxide or aqueous ammonia. As an embodiment of the present disclosure, a concentration of the aqueous ammonia may be 0.1-15.0 M, and specifically 0.1 M, 1 M, 5 M, 10 M, 11 M, 12 M, 13 M, 14 M or 15 M. In an embodiment, an amount ratio of the lanthanum salt and/or the lanthanum salt hydrate to the aqueous ammonia may be in a range of 1 g : 0.5-10 mL, and specifically 1 g : 0.6 mL, 1 g : 0.7 mL, 1 g : 3 mL, 1 g : 6 mL or 1 g : 8 mL. As an embodiment of the present disclosure, the alkali metal hydroxide may include potassium hydroxide, sodium hydroxide, or lithium hydroxide. In an embodiment, a concentration of the alkali reagent in the system during the metathesis reaction may be in a range of 0.1-15.0 M, and specifically 0.1 M, 0.5 M, 0.8 M, 1 M, 1.2 M, 1.5 M, 2 M, 5 M, 10 M or 15 M. In an embodiment, a mass ratio of the lanthanum salt and/or the lanthanum salt hydrate to the alkali metal hydroxide may be in a range of 1 : 0.2-5, specifically 1 : 0.3, 1 : 0.5, 1 : 1, 1 : 3 or 1 : 5.

As an embodiment of the present disclosure, the metathesis reaction may be performed at a temperature of 0-200°C, further 20-150°C, and specifically 25°C, 30°C, 50°C, 80°C, 100°C or 120°C. In an embodiment, the metathesis reaction may be performed at a pressure of 0.1-2.0 MPa, i.e., atmospheric pressure or high pressure, and specifically 0.2 MPa, 0.5 MPa or 1.0 MPa. In an embodiment, the metathesis reaction may be performed for 1-24 h, and specifically 2 h, 4 h, 8 h, 12 h or 16 h. As an embodiment of the present disclosure, after the metathesis reaction, the method may further include: subjecting a lanthanum hydroxide colloid solution obtained after the metathesis reaction to solid-liquid separation, collecting a resulting solid, and sequentially washing and drying the resulting solid to obtain lanthanum hydroxide and/or a lanthanum hydroxide hydrate. As an embodiment of the present disclosure, the solid-liquid separation may be performed by filtration or centrifugation. In an embodiment, a reagent used for the washing may be water, and specifically deionized water. In an embodiment, the drying may be performed at a temperature of 30-150°C, further 80-120°C, and specifically 100-110°C. In an embodiment, the drying may be performed for 1-10 h, further 2-8 h, and specifically 3-5 h. As an embodiment of the present disclosure, reference may be made to the above method I for conditions of the calcination, which are not repeated here.

### Method III:

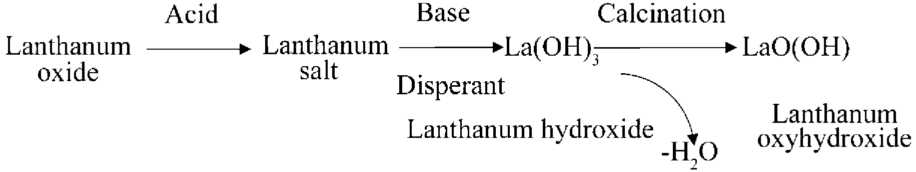

In the present disclosure, a dispersant, an alkali reagent, water, and a lanthanum salt and/or a lanthanum salt hydrate are mixed, and a resulting mixture is subjected to metathesis reaction to obtain lanthanum hydroxide and/or a lanthanum hydroxide hydrate; and the lanthanum hydroxide and/or the lanthanum hydroxide hydrate are calcined to obtain the lanthanum oxyhydroxide.

As an embodiment of the present disclosure, the dispersant may include polyethylene glycol, a surfactant, or an ionic liquid. In an embodiment, the polyethylene glycol may be PEG400, PEG600 or PEG20000. In an embodiment, the surfactant may be polyvinylpyrrolidone or cetyltrimethylammonium bromide. In an embodiment, the ionic liquid may be an N-methyl-N-benzylmorpholinium ionic liquid. In an embodiment, a mass of the dispersant may be in a range of 0.5-10.0 wt%, specifically 0.5 wt%, 1.0 wt%, 1.5 wt%, 2.0 wt%, 2.5 wt%, 3.0 wt%, 3.5 wt%, 4.0 wt%, 4.5 wt%, 5.0 wt%, 8.0 wt% or 10 wt% of the total mass of the lanthanum raw material (i.e., a lanthanum salt and/or a lanthanum salt hydrate). As an embodiment of the present disclosure, reference may be made to the above method II for the type and amount of the lanthanum salt, the type and amount of the alkali reagent, conditions for the metathesis reaction, and conditions for the calcination, which are not repeated here.

### Method IV:

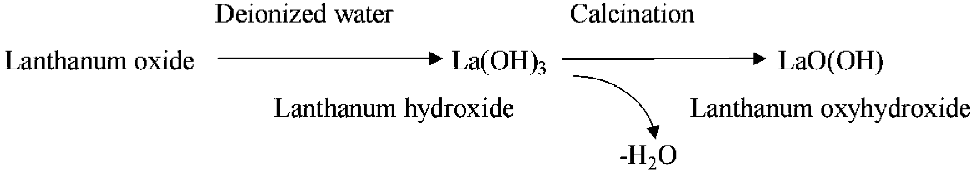

In the present disclosure, lanthanum oxide is mixed with water, and a resulting mixture is subjected to combination reaction to obtain lanthanum hydroxide and/or lanthanum hydroxide hydrate; and the lanthanum hydroxide and/or lanthanum hydroxide hydrate are calcined to obtain the lanthanum oxyhydroxide.

In an embodiment of the present disclosure, the water may be deionized water. In an embodiment, an amount ratio of the lanthanum oxide to water may be 1 g : 10-30 mL, and specifically 1 g : 10 mL, 1 g : 15 mL, 1 g : 20 mL, 1 g : 25 mL or 1 g : 30 mL. As an embodiment of the present disclosure, the combination reaction may be performed at a temperature of 0-200°C, further 20-150°C, and specifically 40°C, 50°C, 60°C, 80°C, 90°C, 100°C, 110°C or 130°C. In an embodiment, the combination reaction is performed at a pressure of 0.1-2.0 MPa, i.e., atmospheric pressure or high pressure, which is not specially limited in the present disclosure. In an embodiment, the combination reaction is performed for 1-24 h, and specifically 2 h, 4 h, 8 h, 12 h or 16 h. As an embodiment of the present disclosure, after the combination reaction, the method may further include: leaving the lanthanum hydroxide colloidal solution obtained after the combination reaction to stand and then performing solid-liquid separation, collecting a resulting solid, and sequentially washing and drying the resulting solid to obtain the lanthanum hydroxide and/or a lanthanum hydroxide hydrate (specifically lanthanum hydroxide and/or lanthanum hydroxide hydrate). As an embodiment of the present disclosure, the solid-liquid separation may be performed by filtration or centrifugation. In an embodiment, a reagent used for the washing may be water, and specifically deionized water. In an embodiment, the drying may be performed at a temperature of 30-150°C, further 80-120°C, and specifically 100-110°C. In an embodiment, the drying may be performed for 1-10 h, further 2-8 h, and specifically 3-5 h. As an embodiment of the present disclosure, reference may be made to the above method I for conditions of the calcination, which are not repeated here.

### Method V:

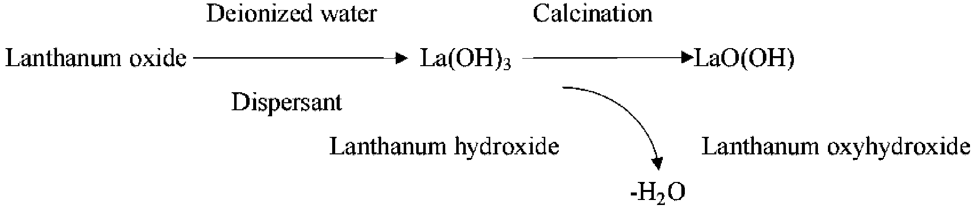

In the present disclosure, lanthanum oxide, a dispersant, and water are mixed, and a resulting mixture is subjected to combination reaction to obtain lanthanum hydroxide and/or a lanthanum hydroxide hydrate (specifically lanthanum hydroxide and/or a lanthanum hydroxide hydrate); and the lanthanum hydroxide and/or the lanthanum hydroxide hydrate are calcined to obtain the lanthanum oxyhydroxide.

As an embodiment of the present disclosure, the dispersant may include polyethylene glycol, a surfactant or an ionic liquid. In an embodiment, the polyethylene glycol may be PEG400, PEG600 or PEG20000. In an embodiment, the surfactant may be polyvinylpyrrolidone or cetyltrimethylammonium bromide. In an embodiment, the ionic liquid may be an N-methyl-N-benzylmorpholinium ionic liquid. In an embodiment, a mass of the dispersant may be 0.5-5.0 wt%, specifically 0.5 wt%, 1.0 wt%, 1.5 wt%, 2.0 wt%, 2.5 wt%, 3.0 wt%, 3.5 wt%, 4.0 wt%, 4.5 wt% or 5.0 wt% of the mass of lanthanum oxide. As an embodiment of the present disclosure, reference may be made to the above method IV for the amount ratio of the lanthanum oxide to water, conditions for the combination reaction, and conditions for the calcination, which are not repeated here.

The present disclosure provides a drug for treating hyperphosphatemia or a disease associated with hyperphosphatemia, including: an active ingredient and a pharmaceutically acceptable adjuvant, where the active ingredient is lanthanum oxyhydroxide.

The present disclosure provides a drug for treating hyperphosphatemia or a disease associated with hyperphosphatemia, including: an active ingredient and a pharmaceutically acceptable adjuvant, where the active ingredient includes at least one of: a lanthanum oxyhydroxide solvate; lanthanum oxyhydroxide containing a lanthanum isotope and/or an oxygen isotope; and a double salt containing lanthanum oxyhydroxide. As an embodiment of the present disclosure, the lanthanum oxyhydroxide solvate may include a lanthanum oxyhydroxide hydrate and/or lanthanum oxyhydroxide organic solvate.

As an embodiment of the present disclosure, a content of the active ingredient in the drug may be in a range of 0.1-99.9 wt%. In the present disclosure, there is no special limitation in the type of the pharmaceutically acceptable adjuvant.

As an embodiment of the present disclosure, a dosage form of the drug may include a granule, a tablet, a capsule, a chewable tablet, a powder, a suspension, or a dry suspension.

The present disclosure provides a method for treating hyperphosphatemia or a disease associated with hyperphosphatemia, including: administering an effective amount of an active ingredient to a patient suffering from hyperphosphatemia or a disease associated with hyperphosphatemia, where the active ingredient is lanthanum oxyhydroxide.

The present disclosure provides a method for treating hyperphosphatemia or a disease associated with hyperphosphatemia, including: administering an effective amount of an active ingredient to a patient suffering from hyperphosphatemia or a disease associated with hyperphosphatemia, where the active ingredient includes at least one of: a lanthanum oxyhydroxide solvate; lanthanum oxyhydroxide containing a lanthanum isotope and/or an oxygen isotope; and a double salt containing lanthanum oxyhydroxide.

The present disclosure provides a lanthanum oxyhydroxide that meets pharmaceutical standards. By means of *in-vitro* lanthanum ion dissolution and release experiments, lanthanum-phosphorus binding experiments, and *in-vivo* animal efficacy experiments, it is proved that the lanthanum oxyhydroxide can be developed as a drug for treating hyperphosphatemia and a disease associated with hyperphosphatemia in humans. In addition, the lanthanum oxyhydroxide provided in the present disclosure does not exhibit any adverse effects in the *in-vivo* efficacy experiments in animals at a therapeutic dose, and no toxicological reactions are observed, indicating that the lanthanum oxyhydroxide exhibits good safety and good development potential and value.

The technical solutions in the present disclosure will be described clearly and completely below with reference to the examples of the present disclosure. Apparently, the described examples are merely some rather than all of the examples of the present disclosure. Based on the examples of the present disclosure, all other examples that would have been obtained by those of ordinary skill in the art without any creative efforts shall fall within the scope of protection of the present disclosure.

### Example 1

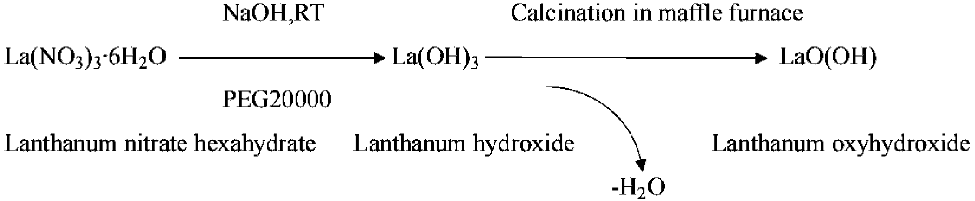

Lanthanum nitrate hexahydrate (17.32 g) was weighed and dissolved in 50 mL of deionized water. Water was then added to up to a total volume of 100 mL to obtain a lanthanum nitrate solution (0.4 mol/L).

Sodium hydroxide (4.8 g) was weighed and dissolved in deionized water, and a resulting mixture was then cooled to room temperature. Water was then added to up to a total volume of 100 mL to obtain a sodium hydroxide solution (1.2 mol/L).

PEG20000 (0.2598 g, with an addition amount of 1.5 wt% of the mass of lanthanum nitrate hexahydrate) was added to the lanthanum nitrate solution. A resulting mixture was cooled to 10°C in an ice-water bath, and the sodium hydroxide solution was then added dropwise thereto. After the dropwise addition was completed, a resulting mixture was stirred for reaction at room temperature (25°C) for 3 h. A resulting reaction mixture was filtered, and a resulting filter cake was washed 3 times with deionized water, and then dried in an oven at 110°C for 3 h to obtain a lanthanum hydroxide solid powder. 10 g of the lanthanum hydroxide solid powder was taken and placed into a muffle furnace and calcined at 420°C for 8 h under a nitrogen atmosphere to obtain 9.10 g of lanthanum oxyhydroxide.

### Examples 2-11

Lanthanum oxyhydroxide was prepared according to the method of Example 1, except that at least one of the following was different: starting materials used, reaction conditions, and calcination conditions, with details listed in Table 1, and unmentioned conditions were the same as those in Example 1.

**Table 1 Conditions and results for the preparation of lanthanum oxyhydroxide in Examples 1-11**

| Example No. | Type and amount of lanthanum source | Type of dispersant and mass percentage relative to lanthanum source | Type and amount of alkali reagent | Reaction condition | Calcination condition | Product yield |
|---|---|---|---|---|---|---|
| 1 | Lanthanum nitrate hexahydrate 17.32 g | PEG20000; 1.5 wt% | Sodium hydroxide 4.8 g | 25°C; 3 h | 420°C; 8 h | 9.10 g |
| 2 | Lanthanum nitrate hexahydrate 17.32 g | / | Sodium hydroxide 4.8 g | 25°C; 3 h | 420°C; 8 h | 9.12 g |
| 3 | Lanthanum nitrate hexahydrate 17.32 g | PEG20000; 1.5 wt% | Sodium hydroxide 4.8 g | 80°C; 2 h | 420°C; 8 h | 9.12 g |
| 4 | Lanthanum nitrate hexahydrate 17.32 g | / | Sodium hydroxide 4.8 g | 80°C; 2 h | 420°C; 8 h | 9.09 g |
| 5 | Lanthanum nitrate hexahydrate 17.32 g | / | Sodium hydroxide 4.8 g | 80°C; 2 h | 350°C; 16 h | 9.08 g |
| 6 | Lanthanum nitrate hexahydrate 17.32 g | / | Sodium hydroxide 4.8 g | 80°C; 2 h | 330°C; 16 h | 9.10 g |
| 7 | Lanthanum nitrate hexahydrate 17.32 g | / | 13 M aqueous ammonia 10 mL | 25°C; 2 h | 420°C; 8 h | 9.10 g |
| 8 | Lanthanum nitrate hexahydrate 17.32 g | / | 13 M aqueous ammonia 10 mL | 80°C; 2 h | 420°C; 8 h | 9.12 g |
| 9 | Lanthanum nitrate hexahydrate 17.32 g | / | Sodium hydroxide 4.8 g | 120°C; 0.2 MPa; 2 h (autoclave) | 400°C; 16 h | 9.09 g |
| 10 | Lanthanum chloride heptahydrate 14.85 g | / | Sodium hydroxide 4.8 g | 80°C; 2 h | 400°C; 16 h | 9.13 g |
| 11 | Lanthanum chloride heptahydrate 14.85 g | / | 13 M aqueous ammonia 10 mL | 80°C; 2 h | 450°C; 8 h | 9.12 g |

### Example 12

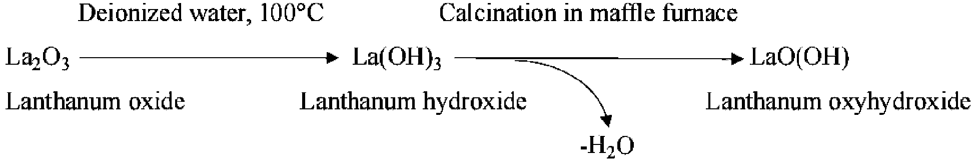

10.0 g of lanthanum oxide was weighed, and 200 mL of deionized water was added. A resulting mixture was heated to reflux temperature (100°C) and stirred for reaction for 12 h, and a resulting reaction mixture was then cooled naturally, left to stand and filtered. A resulting filter cake was washed 3 times with deionized water, and then dried in an oven at 110°C for 3 h to obtain a lanthanum hydroxide solid powder. 10 g of the lanthanum hydroxide solid powder was taken and placed into a muffle furnace and calcined at 450°C for 8 h under a nitrogen atmosphere to obtain 9.10 g of lanthanum oxyhydroxide.

### Example 13

This example was performed according to the method of Example 12, except that the reaction temperature was 50°C and finally 9.08 g of lanthanum oxyhydroxide was obtained.

### Example 14

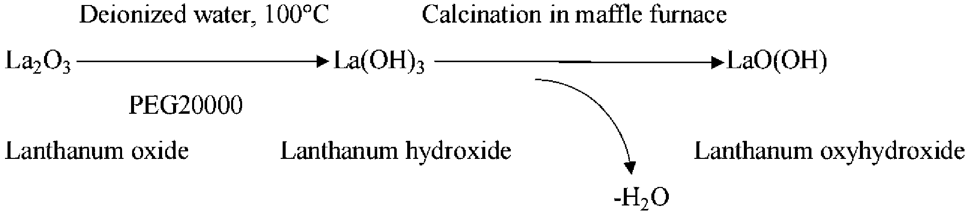

10.0 g of lanthanum oxide was weighed, and 200 mL of deionized water and 0.15 g of PEG20000 were added thereto. A resulting mixture was heated to reflux temperature (100°C) and stirred for reaction for 12 h. A resulting reaction mixture was then cooled naturally, left to stand and filtered. A resulting filter cake was washed 3 times with deionized water, and then dried in an oven at 110°C for 3 h to obtain a lanthanum hydroxide solid powder. 10 g of the lanthanum hydroxide solid powder was taken and placed into a muffle furnace and calcined at 450°C for 24 h under a nitrogen atmosphere to obtain 9.13 g of lanthanum oxyhydroxide.

### Example 15

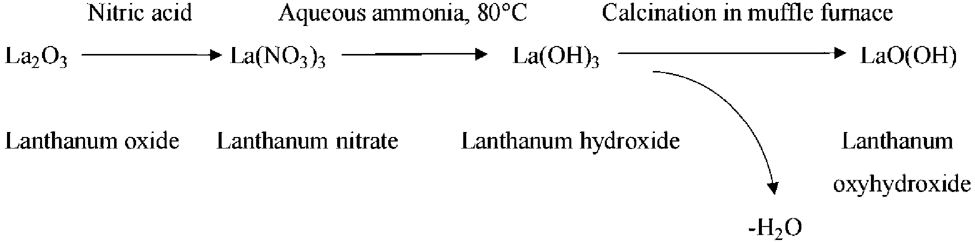

178 g of concentrated nitric acid at a concentration of 67 wt% was weighed and added to a beaker containing 500 mL of deionized water. Deionized water was then added to up to a total volume of 2 L. 100 g of lanthanum oxide was added thereto in batches. A resulting mixture was stirred at room temperature for 1 h to obtain a clear lanthanum nitrate solution.

50 mL of concentrated aqueous ammonia at a concentration of 13 M was measured, and deionized water was added to up to a total volume of 650 mL to obtain aqueous ammonia at a concentration of 1 M.

500 mL of the lanthanum nitrate solution was measured and 325 mL of the aqueous ammonia at a concentration of 1 M was added dropwise thereto at room temperature. After the dropwise addition was completed, a resulting mixture was stirred for reaction at 80°C for 2 h. A resulting reaction mixture was then cooled and filtered. A resulting filter cake was washed 3 times with deionized water and dried in an oven at 110°C for 3 h to obtain a lanthanum hydroxide solid powder. 10 g of the lanthanum hydroxide solid powder was taken and placed into a muffle furnace and calcined at 380°C for 8 h under a nitrogen atmosphere to obtain 9.08 g of lanthanum oxyhydroxide.

### Example 16

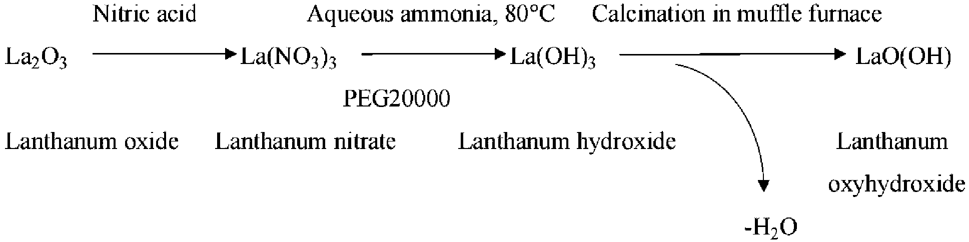

178 g of concentrated nitric acid at a concentration of 67 wt% was weighed and added to a beaker containing 500 mL of deionized water. Deionized water was then added to up to a total volume of 2 L. 100 g of lanthanum oxide was added thereto in batches. A resulting mixture was stirred at room temperature for 1 h to obtain a clear lanthanum nitrate solution.

50 mL of concentrated aqueous ammonia at a concentration of 13 M was measured, and deionized water was added to up to a total volume of 650 mL to obtain aqueous ammonia at a concentration of 1 M.

500 mL of the lanthanum nitrate solution was measured and 325 mL of the aqueous ammonia at a concentration of 1 M was added dropwise thereto at room temperature. After the dropwise addition was completed, 5.0 g of PEG20000 was added thereto. A resulting mixture was heated to 80°C and stirred for reaction at 80°C for 2 h. A resulting reaction mixture was then cooled and filtered. A resulting filter cake was washed 3 times with deionized water and dried in an oven at 110°C for 3 h to obtain a lanthanum hydroxide solid powder. 10 g of the lanthanum hydroxide solid powder was taken and placed into a muffle furnace and calcined at 380°C for 16 h under a nitrogen atmosphere to obtain 9.08 g of lanthanum oxyhydroxide.

### Example 17

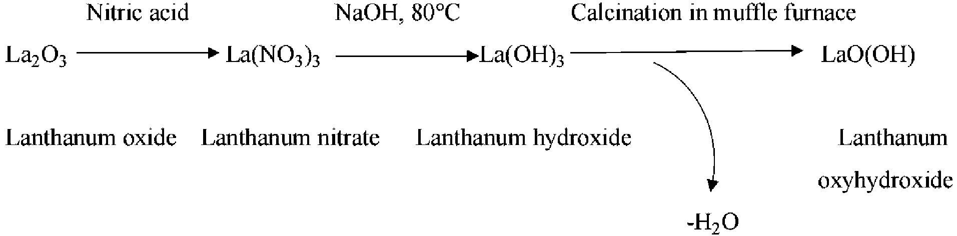

178 g of concentrated nitric acid at a concentration of 67 wt% was weighed and added to a beaker containing 500 mL of deionized water. Deionized water was then added to up to a total volume of 2 L. 100 g of lanthanum oxide was added thereto in batches. A resulting mixture was stirred at room temperature for 1 h to obtain a clear lanthanum nitrate solution.

10 g of sodium hydroxide was weighed and dissolved in deionized water, and a resulting mixture was then cooled to room temperature. Water was then added to up to a total volume of 250 mL to obtain a sodium hydroxide solution at a concentration of 1 M.

500 mL of the lanthanum nitrate solution was measured and 250 mL of the sodium hydroxide solution was added dropwise thereto at room temperature. After the dropwise addition was completed, a resulting mixture was heated to 80°C and stirred for reaction at 80°C for 3 h. A resulting reaction mixture was then cooled and filtered. A resulting filter cake was washed 3 times with deionized water and dried in an oven at 110°C for 3 h to obtain a lanthanum hydroxide solid powder. 10 g of the lanthanum hydroxide solid powder was taken and placed into a muffle furnace, and calcined at 420°C for 8 h under a nitrogen atmosphere to obtain 9.09 g of lanthanum oxyhydroxide.

### Example 18

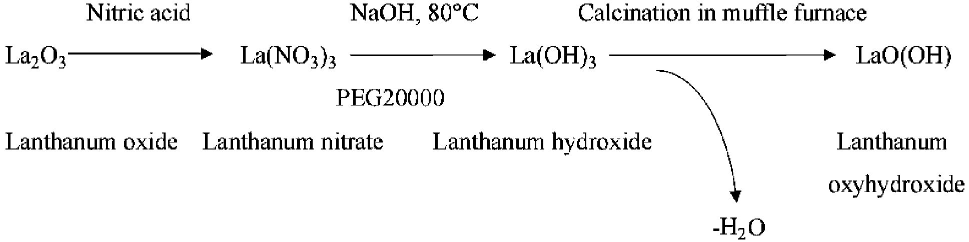

178 g of concentrated nitric acid at a concentration of 67 wt% was weighed and added to a beaker containing 500 mL of deionized water. Deionized water was then added to up to a total volume of 2 L. 100 g of lanthanum oxide was added thereto in batches. A resulting mixture was stirred at room temperature for 1 h to obtain a clear lanthanum nitrate solution.

10 g of sodium hydroxide was weighed and dissolved in deionized water, and a resulting mixture was then cooled to room temperature. Water was then added to up to a total volume of 250 mL to obtain a sodium hydroxide solution at a concentration of 1 M.

500 mL of the lanthanum nitrate solution was measured and 250 mL of the sodium hydroxide solution was added dropwise thereto at room temperature. After the dropwise addition was completed, 5.0 g of PEG20000 was added thereto. A resulting mixture was heated to 80°C and stirred for reaction at 80°C for 3 h. A resulting reaction mixture was then cooled and filtered. A resulting filter cake was washed 3 times with deionized water and dried in an oven at 110°C for 3 h to obtain a lanthanum hydroxide solid powder. 10 g of the lanthanum hydroxide solid powder was taken and placed into a muffle furnace and calcined at 420°C for 8 h under a nitrogen atmosphere to obtain 9.10 g of lanthanum oxyhydroxide.

The lanthanum oxyhydroxide prepared in Examples 1-18 was subjected to X-ray diffraction (XRD) test. The resulting XRD patterns were consistent with the corresponding standard reference cards (PDF files).

### Test Example Dissolution experiments of lanthanum oxyhydroxide and lanthanum carbonate in different media

In this test example, the dissolution of lanthanum ions in solutions at different time points in different media was investigated, and the release rates of lanthanum ions in different media were compared, specifically as follows:
Investigated media: a hydrochloric acid solution (pH = 1.2), an acetate buffer (pH = 3.0), and an acetate buffer (pH = 5.0).

Dissolution condition: each medium at a temperature of 37°C; 100 rpm; a volume of 900 mL; sampling point at 0.5 h, 1 h, 1.5 h, 2 h, and 3 h (no replenishment).

### Preparation Process:

Lanthanum oxyhydroxide: About 0.62 g of the product (in terms of lanthanum element: 500 mg, specifically lanthanum oxyhydroxide prepared in Example 12, sieved through a 60-mesh sieve prior to the experiment) was taken and placed in a dissolution apparatus. A test was performed according to the specified dissolution conditions. Samples were collected at specified time points and filtered, and a resulting filtrate was collected for analytical detection.

Lanthanum carbonate: About 0.953 g of the product (in terms of lanthanum: 500 mg; batch number: TSL-240622; sieved through a 60-mesh sieve prior to the experiment) was taken and placed in a dissolution apparatus. A test was performed according to the specified dissolution conditions. Samples were collected at specified time points and filtered, and a resulting filtrate was collected for analytical detection.

Test method: 16.4 g of anhydrous sodium acetate was taken, and dissolved in 1000 mL of water, and a pH value of a resulting mixture was adjusted to 6.2 with glacial acetic acid to obtain a 0.2 mol/L acetate buffer. 5 mL of the above filtrate was precisely measured and placed in an Erlenmeyer flask. 45 mL of the 0.2 mol/L acetate buffer and 5-6 drops of xylenol orange indicator solution were added thereto. Titration was performed with ethylenediaminetetraacetic acid disodium titrant (0.01 mol/L) until the solution changed from pale purple to lemon yellow. Each 1 mL of the ethylenediaminetetraacetic acid disodium titrant was equivalent to 1.39 mg of La, and the lanthanum content in the filtrate was calculated based on the amount of the ethylenediaminetetraacetic acid disodium titrant used.

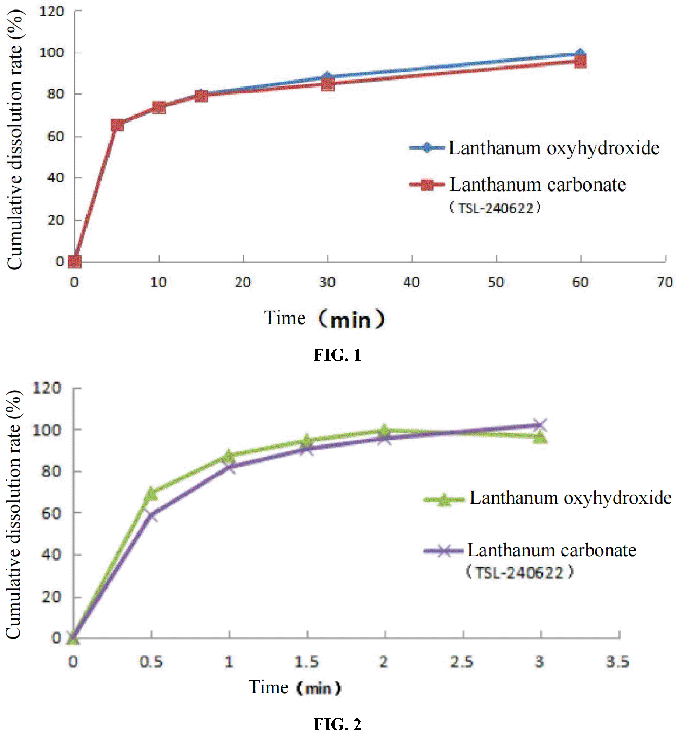

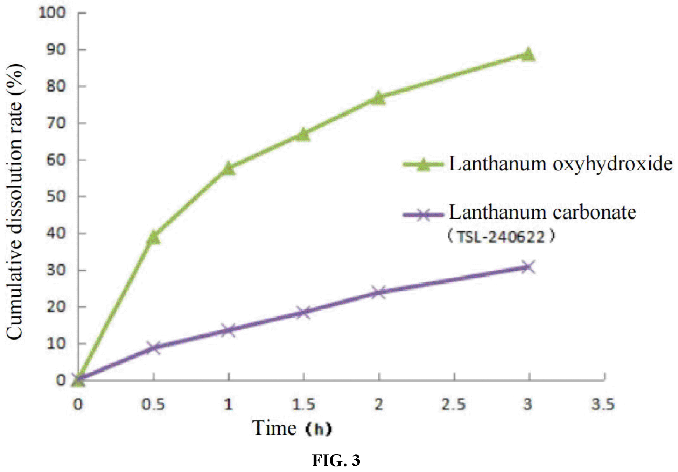

FIG. 1 shows the dissolution curves of lanthanum oxyhydroxide and lanthanum carbonate in a medium of hydrochloric acid solution with a pH value of 1.2, FIG. 2 shows the dissolution curves of lanthanum oxyhydroxide and lanthanum carbonate in a medium of acetate buffer with a pH value of pH 3.0, and FIG. 3 shows the dissolution curves of lanthanum oxyhydroxide and lanthanum carbonate in a medium of acetate buffer with a pH value of pH 5.0. Specific data are listed in Tables 2-4.

**Table 2 Cumulative dissolution rates (%) of lanthanum oxyhydroxide and lanthanum carbonate in a medium of hydrochloric acid solution with a pH value of 1.2**

| Hydrochloric acid solution medium with a pH value of 1.2 (100 rpm) | | | | | | |
|---|---|---|---|---|---|---|
| Time (min) | 0.0 | 5 | 10 | 15 | 30 | 60 |
| Lanthanum oxyhydroxide | 0.0 | 64.9 | 73.6 | 79.6 | 87.9 | 99.0 |
| Lanthanum carbonate (TSL-240622) | 65.3 | 73.7 | 79.1 | 84.7 | 95.6 | 65.3 |

**Table 3 Cumulative dissolution rates (%) of lanthanum oxyhydroxide and lanthanum carbonate in a medium of acetate buffer with a pH value of 3.0**

| Acetate solution medium with a pH value of 3.0 (shaking at 100 rpm) | | | | | | |
|---|---|---|---|---|---|---|
| Time (h) | 0 | 0.5 | 1 | 1.5 | 2 | 3 |
| Lanthanum oxyhydroxide | 0.0 | 69.4 | 87.3 | 94.5 | 99.5 | 96.7 |
| Lanthanum carbonate (TSL-240622) | 0.0 | 58.7 | 81.7 | 90.5 | 95.6 | 102.0 |

**Table 4 Cumulative dissolution rates (%) of lanthanum oxyhydroxide and lanthanum carbonate in a medium of acetate buffer with a pH value of 5.0**

| Acetate solution medium with a pH value of 5.0 (shaking at 100 rpm) | | | | | | |
|---|---|---|---|---|---|---|
| Time (h) | 0 | 0.5 | 1 | 1.5 | 2 | 3 |
| Lanthanum oxyhydroxide | 0.0 | 38.9 | 57.6 | 66.8 | 76.7 | 88.6 |
| Lanthanum carbonate (TSL-240622) | 0.0 | 8.6 | 13.3 | 18.2 | 23.7 | 30.7 |

As could be seen from the above dissolution data and dissolution curves in media at different pH values, the dissolution rates of lanthanum oxyhydroxide and lanthanum carbonate are substantially the same in the dissolution media with pH values of 1.2 and pH 3.0. However, when the pH is increased to 5.0, both the dissolution rate and cumulative dissolution rate of lanthanum oxyhydroxide are significantly better than those of the control drug lanthanum carbonate. This indicates that the dissolution of the control drug lanthanum carbonate is more affected by the pH value. Moreover, lanthanum-containing pharmaceutical products generally need to be taken with food or after a meal. Since the pH in the human body generally rises to 3-5 after a meal, it can be presumed that when lanthanum carbonate and lanthanum oxyhydroxide are respectively taken after a meal in human efficacy trials, the phosphorus-lowering rate and phosphorus-lowering amount of lanthanum oxyhydroxide may be superior to those of the control drug lanthanum carbonate. This suggests that pharmaceutical formulations of lanthanum oxyhydroxide have better efficacy and potential value in lowering serum phosphorus and treating related diseases such as hyperphosphatemia and chronic kidney disease in humans.

### Test Example Pharmacodynamic evaluation of lanthanum oxyhydroxide for treating hyperphosphatemia induced by chronic renal failure in SD rats

By establishing a model of chronic renal failure with hyperphosphatemia in male SD rats induced by 5/6 nephrectomy, and using lanthanum carbonate as a positive control drug, the phosphorus-lowering effect and renal function-improving effect of different doses of lanthanum oxyhydroxide on model animals were investigated, and the pharmacodynamic characteristics thereof in the treatment of hyperphosphatemia were comprehensively evaluated. The specific test was as follows:
Male SD rats, 6 weeks old, purchased from SPF (Beijing) biotechnology Co. Ltd., China; license number: SYXK (Beijing) 2023-0004; and certificate number: No. 110324251101765076.
Test drug source:
   Lanthanum carbonate: Batch No. TSL-241253, Beijing Horicin Biotechnology Co., Ltd., China;
   Lanthanum oxyhydroxide: Batch No. HRL1101-25031401, Beijing Horicin Biotechnology Co., Ltd., China;
   Vehicle source:
      Sodium carboxymethylcellulose (CMC-Na): Batch No. JS241201, Jiangxi Alpha Hi-tech Pharmaceutical Co., Ltd., China;
      Corn oil: Batch No. 01033, Shandong Luhua Group Co., Ltd, China.

(1) Establishment of animal model: Male SD rats underwent surgical resection of the entire right kidney and approximately 2/3 of the left kidney (with the adrenal gland preserved), resulting in an approximate 5/6 reduction in the total volume of functional renal tissue. All animals were fed with a high-phosphorus diet (phosphorus content: 1.2 wt%) after 1 week of postoperative recovery. After 7 consecutive weeks of feeding with the high-phosphorus diet, using significantly elevated serum phosphorus, creatinine, and urea nitrogen levels as core modeling indicators, successfully-modeled rats with similar serum phosphorus levels, together with sham-operated rats (where only renal capsule was separated without renal tissue resection) tested in the same period, were used as experimental animals. The selected model rats were randomly assigned to administration groups, and the serum phosphorus, creatinine, and urea nitrogen data at this stage were recorded as baseline values before drug administration.
(2) Preparation of formulation for administration: The desired amount of test drug was weighed, and a small amount of corn oil solution containing 1 wt% CMC-Na was added into the mortar. A resulting mixture was ground slowly for 10 min, and then transferred. The mortar was rinsed repeatedly with small portions of the corn oil solution containing 1 wt% CMC-Na. A resulting solution was brought to the desired volume, and the resulting solution was eddied for uniform mixing.
(3) Grouping and administration: Animals were selected with serum phosphorus, creatinine, and urea nitrogen levels as indicators for being successfully-selected and also as baseline data. The animals selected were randomly divided into the following groups (12 rats per group): model group, lanthanum carbonate group (1000 mg/kg, lanthanum content: about 500 mg/kg), low-dose lanthanum oxyhydroxide group (200 mg/kg, lanthanum content: about 167 mg/kg), medium-dose lanthanum oxyhydroxide group (400 mg/kg, lanthanum content: about 330 mg/kg), and high-dose lanthanum oxyhydroxide group (600 mg/kg, lanthanum content: about 500 mg/kg). Additionally, 12 sham-operated rats without renal resection were selected as the control group. Animals in control group and model group were fed a high-phosphorus diet and subjected to continuous oral gavage of the vehicle once daily. Animals in lanthanum carbonate group, and low-dose, medium-dose and high-dose lanthanum oxyhydroxide groups were subjected to continuous oral gavage of the corresponding drug suspensions once daily (administration volume: 5 mL/kg for all groups). Blood samples were collected to detect serum phosphorus at week 4 and week 6 after administration. At the end of the experiment at week 6, animals in each group were dissected, and 7 kidneys were collected from each group for HE staining to evaluate the degree of renal injury.

**Table 5 Grouping of experimental animals**

| Group | Number of animals (rats) | Drug name | Administration dose (in terms of compound, mg/kg) | Administration volume (mL/kg) | Administration route |
|---|---|---|---|---|---|
| Group 1 - Control group | 12 | Vehicle | - | 5 | Gavage |
| Group 2 - Model group | 12 | Vehicle | - | 5 | Gavage |
| Group 3 - Lanthanum carbonate group | 12 | Lanthanum carbonate | 1000 | 5 | Gavage |
| Group 4 - Low-dose lanthanum oxyhydroxide group | 12 | Lanthanum oxyhydroxide | 200 | 5 | Gavage |
| Group 5 - Medium-dose lanthanum oxyhydroxide group | 12 | Lanthanum oxyhydroxide | 400 | 5 | Gavage |
| Group 6 - High-dose lanthanum oxyhydroxide group | 12 | Lanthanum oxyhydroxide | 600 | 5 | Gavage |

(4) Test results: The results in Table 6 show that at week 6 after administration, the serum phosphorus levels in the medium-dose lanthanum oxyhydroxide group, high-dose lanthanum oxyhydroxide group and lanthanum carbonate group are significantly reduced compared with the model group (p < 0.05, p < 0.001, p < 0.01, respectively). This indicates that both lanthanum oxyhydroxide and lanthanum carbonate can significantly lower the serum phosphorus level in model rats, and show a mild alleviating trend in improving the renal function (with creatinine and urea nitrogen as indicators) and renal pathological damage of model rats.

**Table 6 Statistical table of serum test data after 6 weeks of administration**

| Group | Administration dose (in terms of compound, mg/kg) | Mean ± SD | | |
|---|---|---|---|---|
| | | Phosphorus (mmol/L) | Creatinine (µmol/L) | Urea nitrogen (mmol/L) |
| Group 1 - Control group | - | 2.05 ± 0.17 | 27.9 ± 1.88 | 4.5 ± 0.72 |
| Group 2 - Model group | - | 2.93 ± 1.06^{#} | 127.1 ± 42.71^{###} | 19.1 ± 7.78^{###} |
| Group 3 - Lanthanum carbonate group | 1000 | 1.91 ± 0.26** | 121.3 ± 43.96 | 15.4 ± 6.42 |
| Group 4 - Low-dose lanthanum oxyhydroxide group | 200 | 2.26 ± 0.56 | 116.8 ± 52.68 | 14.5 ± 5.15 |
| Group 5 - Medium-dose lanthanum oxyhydroxide group | 400 | 2.17 ± 0.33* | 114.8 ± 42.14 | 14.1 ± 4.64 |
| Group 6 - High-dose lanthanum oxyhydroxide group | 600 | 1.59 ± 0.34** | 103.9 ± 32.71 | 11.9 ± 2.72 |

| | | | | |
|---|---|---|---|---|
| Note: in Table 6, * represents *p* < 0.05, ** represents *p* < 0.01 *vs* model group; # represents *p* < 0.05, ### represents *p* < 0.001 *vs* control group. | | | | |

The foregoing descriptions are merely preferred embodiments of the present disclosure, and it should be noted that for those of ordinary skill in the art, without departing from the principles of the present disclosure, several improvements and refinements may also be made, which should also be deemed as falling within the scope of the present disclosure.

## Claims

1. Use of lanthanum oxyhydroxide in preparation of a drug for treating hyperphosphatemia or a disease associated with hyperphosphatemia.

2. Use of at least one selected from the group consisting of lanthanum oxyhydroxide containing a lanthanum isotope and/or an oxygen isotope, a lanthanum oxyhydroxide solvate, and a double salt containing lanthanum oxyhydroxide in preparation of a drug for treating hyperphosphatemia or a disease associated with hyperphosphatemia.

3. The use as claimed in claim 1 or 2, wherein the disease associated with hyperphosphatemia comprises chronic kidney disease, renal failure, secondary hyperparathyroidism, metabolic bone disease, soft tissue calcification, or cardiovascular calcification.

4. The use as claimed in claim 1 or 2, wherein the lanthanum oxyhydroxide is in a form of a powder.

5. The use as claimed in claim 1 or 2, wherein the lanthanum oxyhydroxide comprises a single crystal form or an amorphous form.

6. The use as claimed in claim 1 or 2, wherein the lanthanum oxyhydroxide has a surface in a smooth or rough state.

7. The use as claimed in claim 1 or 2, wherein the lanthanum oxyhydroxide has a porous structure.

8. The use as claimed in claim 1, wherein the lanthanum oxyhydroxide is prepared by a method comprising one selected from the group consisting of:
method I: calcining lanthanum hydroxide and/or a lanthanum hydroxide hydrate to obtain the lanthanum oxyhydroxide;
method II: mixing an alkali reagent, water, and a lanthanum salt and/or a lanthanum salt hydrate, and subjecting a resulting mixture to metathesis reaction to obtain lanthanum hydroxide and/or a lanthanum hydroxide hydrate; and calcining the lanthanum hydroxide and/or the lanthanum hydroxide hydrate to obtain the lanthanum oxyhydroxide;
method III: mixing a dispersant, an alkali reagent, water, and a lanthanum salt and/or a lanthanum salt hydrate, and subjecting a resultant mixture to metathesis reaction to obtain lanthanum hydroxide and/or a lanthanum hydroxide hydrate; and calcining the lanthanum hydroxide and/or the lanthanum hydroxide hydrate to obtain the lanthanum oxyhydroxide;
method IV: mixing lanthanum oxide with water, and subjecting a resulting mixed material to combination reaction to obtain lanthanum hydroxide and/or a lanthanum hydroxide hydrate; and calcining the lanthanum hydroxide and/or the lanthanum hydroxide hydrate to obtain the lanthanum oxyhydroxide; and
method V: mixing lanthanum oxide, a dispersant, and water, and subjecting an obtained mixture to combination reaction to obtain lanthanum hydroxide and/or a lanthanum hydroxide hydrate; and calcining the lanthanum hydroxide and/or the lanthanum hydroxide hydrate to obtain the lanthanum oxyhydroxide.

9. The use as claimed in claim 8, wherein in the method I, the method II, the method III, the method IV, and the method V, the calcining is performed at a temperature independently of 200-800°C, and the calcining is performed independently for 1-72 hours.

10. The use as claimed in claim 8, wherein in the method II and the method III, the alkali reagent independently comprises an alkali metal hydroxide or aqueous ammonia; and in the method III and the method V, the dispersant independently comprises polyethylene glycol, a surfactant, or an ionic liquid.

11. The use as claimed in claim 10, wherein the aqueous ammonia has a concentration of 0.1-15.0 M; and an amount ratio of the lanthanum salt and/or the lanthanum salt hydrate to the aqueous ammonia is in a range of 1 g : 0.5-10 mL.

12. The use as claimed in claim 10, wherein the alkali metal hydroxide comprises potassium hydroxide, sodium hydroxide, or lithium hydroxide; and a mass ratio of the lanthanum salt and/or the lanthanum salt hydrate to the alkali metal hydroxide is in a range of 1 : 0.2-5.

13. The use as claimed in claim 10, wherein in the method III and the method V, the lanthanum salt and/or the lanthanum salt hydrate is used as a lanthanum raw material, and a mass of the dispersant is 0.5-10.0% of a total mass of the lanthanum raw material.

14. The use as claimed in claim 10 or 13, wherein the polyethylene glycol is PEG400, PEG600, or PEG20000.

15. The use as claimed in claim 10 or 13, wherein the surfactant is polyvinylpyrrolidone or cetyltrimethylammonium bromide.

16. The use as claimed in claim 10 or 13, wherein the ionic liquid is an N-methyl-N-benzylmorpholinium ionic liquid.

17. The use as claimed in claim 8, wherein in the method II and the method III, the metathesis reaction is performed at a temperature independently of 0-200°C and a pressure independently of 0.1-2.0 MPa for a time independently of 1-24 hours.

18. The use as claimed in claim 8, wherein in the method IV and the method V, an amount ratio of the lanthanum oxide to the water is independently in a range of 1 g : 10-30 mL.

19. The use as claimed in claim 8, wherein in the method IV and the method V, the combination reaction is performed at a temperature of 0-200°C and a pressure of 0.1-2.0 MPa for a time of 1-24 hours.

20. A drug for treating hyperphosphatemia or a disease associated with hyperphosphatemia, comprising an active ingredient and a pharmaceutically acceptable adjuvant, wherein the active ingredient is lanthanum oxyhydroxide.

21. A drug for treating hyperphosphatemia or a disease associated with hyperphosphatemia, comprising an active ingredient and a pharmaceutically acceptable adjuvant, wherein the active ingredient comprises at least one selected from the group consisting of: a lanthanum oxyhydroxide solvate, lanthanum oxyhydroxide containing a lanthanum isotope and/or an oxygen isotope, and a double salt containing lanthanum oxyhydroxide.

22. The drug as claimed in claim 20 or 21, wherein a content of the active ingredient in the drug is in a range of 0.1-99.9 wt%.

23. The drug as claimed in claim 20 or 21, wherein the pharmaceutically acceptable adjuvant comprises a pharmaceutically acceptable carrier and/or diluent.

24. The drug as claimed in claim 20 or 21, wherein a dosage form of the drug comprises a granule, a tablet, a capsule, a chewable tablet, a powder, a suspension, or a dry suspension.

25. A method for treating hyperphosphatemia or a disease associated with hyperphosphatemia, comprising: administering an effective amount of an active ingredient to a patient suffering from the hyperphosphatemia or the disease associated with hyperphosphatemia, wherein the active ingredient is lanthanum oxyhydroxide.

26. A method for treating hyperphosphatemia or a disease associated with hyperphosphatemia, comprising: administering an effective amount of an active ingredient to a patient suffering from the hyperphosphatemia or the disease associated with hyperphosphatemia, wherein the active ingredient comprises at least one selected from the group consisting of: a lanthanum oxyhydroxide solvate, lanthanum oxyhydroxide containing a lanthanum isotope and/or an oxygen isotope, and a double salt containing lanthanum oxyhydroxide.
